# EUROPEAN PATENT APPLICATION

(11) **EP 2 036 975 A1**
(43) Date of publication of application: **18.03.2009**
(21) Application number: 07745428.8
(22) Date of filing: 15.06.2007
(51) Int. Cl.: C12M 1/00, C12N 5/02

(54) **MICROCHIP FOR CELL ALIGNMENT AND METHOD OF CELL ALIGNMENT**

(30) Priority: 16.06.2006 JP 2006168013
(71) Applicant: ABsize Inc., Osaka-shi, Osaka 530-0053 (JP)
(72) Inventor: SATO, Setsuya Cybox Co., Ltd., Ibaraki-shi, Osaka 567-0085 (JP); YAMATO, Toshiyuki Cybox Co., Ltd., Ibaraki-shi, Osaka 567-0085 (JP); HAYASHI, Katsuaki Cybox Co., Ltd., Ibaraki-shi, Osaka 567-0085 (JP); MATSUMOTO, Yutaka Cybox Co., Ltd., Ibaraki-shi, Osaka 567-0085 (JP); OCHIYA, Takahiro NATIONAL CANCER CENTER, Chuo-ku, Tokyo 104-0045 (JP); OH, Isamu GRADUATE SCHOOL OF ENGINEERING, Suita-shi, Osaka 565-0871 (JP)
(74) Representative: Skuhra, Udo
(86) International application number: PCT/JP2007/062174
(87) International publication number: WO 2007/145341

(57) **Abstract**

[Problems]

To provide a microchip for cell arranging which transfers and arranges the cell accurately and quickly, by combining advantages of the method of an optical tweezer and of micro channel.

[Means for solving problems]

A microchip for arranging cells, including a base, the base comprising: at least one cell supply channel including a cell inlet port in liquid communication with a supply mechanism configured to supply the cells into the cell supply channel; at least one cell drain channel including a cell drain port in fluid communication with a drain mechanism, the cell drain channel configured to guide the cells to the cell drain port; at least one reservoir for temporarily storing the cells flowing between the cell supply channel and the cell drain channel; at least one cell arrangement portion for receiving cells introduced from the reservoir, the cell arrangement portion comprising plural compartments partitioned by a wall; and at least one junction channel extending from the cell arrangement portion to the reservoir; wherein the reservoir is wider than the cell supply channel, the cell drain channel and the junction channel.

## Description

### BACK GROUND OF THE INVENTION

### FIELD OF THE INVENTION

This invention relates to a microchip and a method for cell arrangement. Further, it relates to a method utilizing liquid flow and optical tweezer both together to transfer cells during an arranging manipulation, and relates to a microchip for cell arrangement used for this method.

### DESCRIPTION OF THE RELATED ART

An optical tweezer manipulation is known as a method of cell transfer in a non contact manner by trapping and scanning a cell with a laser beam radiation pressure (e.g. Patent Reference 1).

This method advantageously provides accurate and free cell transfer but is not efficient due to slow transfer speed which results in a lot of time when transferring numbers of cells or long distances.

Another non-contact method for cell transfer is sending a liquid to a micro channel with micro pump and so on (e.g. Patent Reference 2).

This method has an advantage in transferring the numbers of cells quickly at once. However, it has some difficulties in controlling the amount of cells to be transferred and selecting the specific cells. Further, it is unsuitable for short and accurate movement.

On the other hand, a microchip with a number of microchip cells for cell culture on a base is known as an apparatus for a regular arrangement of cells (e.g. Patent Reference 3).

Such a microchip can be used for making a cell chip to make a pseudo-tissue, which is utilized for a toxicity test or a regenerative medicine for drug discovery purposes by arranging with an arbitrary pattern and cultivating at least two types of cells. Finding the interaction of cells by cultivating different cells contiguously is also effective in the study of cell transfer, adhesion, differentiation, growth and so on.

When such a microchip is used for arranging the cell, the cell is transferred into the microchip cell using an optical tweezer after seeding the cell from the microchip.

However, when the cell seeding was performed from the microchip, an excessive cell was left around the microchip cell for cell culture, which resulted in unreproductive cell chips. Also, it takes a lot of time to transfer and align many cells with the optical tweezer. In this manipulation, and the cells sometimes adhere to the microchip before the transfer or arrangement.

Further, it takes a lot of time to a segregate and extract many cells with the optical tweezer which result in inefficient manipulation due to a slow transfer of the cell, although it is possible to segregate accurately.
[Patent Reference 1]
   Japanese Patent Publication 2001-62792
[Patent Reference 2]
   Japanese Patent Publication 2003-274924
[Patent Reference 3]
   Japanese Patent Publication 2005-27598

### DISCLOSURE OF THE INVENTION

### PROBLEMS TO BE SOLVED BY THE INVENTION

This invention is to solve the above-described problems by combining advantageous aspects of the optical tweezer operation with those of the micro channel method. The method and microchip according to the present invention will provide accurate and quick cell manipulation such as cell transfer and cell arrangement which leads to effective fabrication of cell chips.

### MEANS FOR SOLVING THE PROBLEMS

The invention according to claim 1 relates to a microchip for arranging cells, including a base, the base comprising: at least one cell supply channel including a cell inlet port in liquid communication with a supply mechanism configured to supply the cells into the cell supply channel; at least one cell drain channel including a cell drain port in fluid communication with a drain mechanism, the cell drain channel configured to guide the cells to the cell drain port; at least one reservoir for temporarily storing the cells flowing between the cell supply channel and the cell drain channel; at least one cell arrangement portion for receiving cells introduced from the reservoir, the cell arrangement portion comprising plural compartments partitioned by a wall; and at least one junction channel extending from the cell arrangement portion to the reservoir; wherein the reservoir is wider than the cell supply channel, the cell drain channel and the junction channel.

The invention according to Claim 2 relates to the microchip, wherein an angle at a corner of a flow path through the reservoir from the cell supply channel to the junction channel is a right angle or an acute angle.

The invention according to claim 3 relates to The microchip according to Claim 1 or 2, wherein the cell supply channel is not aligned with the cell drain channel connected to the cell supply channel via the reservoir.

The invention according to claim 4 relates to the microchip according to any one of Claims 1 to 3 comprising plural of the reservoirs, each of reservoir connected to a separate cell supply channel, a separate cell drain channel and a separate junction channel, wherein the separate junction channel terminating at the cell arrangement portion in common.

The invention according to claim 5 relates to the microchip according to any one of Claims 1 to 4, comprising the compartments in a hexagonal shape so as to be arranged in a honeycomb structure, wherein a bottom of each compartment includes a circular aperture smaller than the hexagonal shape.

The invention according to claim 6 relates to a method for arranging cells with the microchip described in any one of Claims 1 to 5, comprising: sequentially repeating a first step of introducing a liquid containing the cells from the supply mechanism into the at least one reservoir via the cell supply channel, a second step of transferring the cells from the reservoir to the cell arrangement portion via the junction channel with a control of the supply mechanism and the drain mechanism or with an optical tweezer, and a third step of placing the cells into the compartments desirably selected in the reservoir with the optical tweezer until a completion of a desired cell arrangement in the cell arrangement portion.

The invention according to claim 7 relates to the method according to Claim 6, further comprising: providing the microchip described in Claim 4 or 5, and providing different types of the cells, wherein the first step includes introducing the cells with sorting by their types so that each reservoir receives different types of cells, and the second step includes transferring a desired cell from each reservoir to the cell arrangement portion via the separate junction channel by controlling the supply mechanism and the drain mechanism or by the optical tweezer.

### EFFECT OF THE INVENTION

The invention according to claim 1 may quickly flow a lot of cells into the reservoir from the cell supply channel by using the supply mechanism connected to the inlet port. Also, a temporal storage of the cell in the reservoir, which results from the wider reservoir than the junction channel, facilitates to provide accurate and desired arrangement of desired cells in the cell compartment of the cell arrangement portion where the desired cells are delivered with the optical tweezer and so on.

Thus, the present invention provides quick and accurate manipulation for cell arrangement.

Also, the liquid flow from the supply mechanism prevents the cell from adhering to any undesired area in the microchip.

Further, undesired cells (e.g. a dead cell or different size cell, etc.) are discharged to the cell drain channel from the reservoir by the drain mechanism connected to the cell drain port.

According to the invention of claim 2, an angle at a corner of a flow path through the reservoir from the cell supply channel to the junction channel is a right angle or an acute angle. This prevents a number of cells from flowing into the cell arrangement portion from the reservoir via the junction channel by the inlet mechanism. Thus, the excessive cell does not flow into the cell arrangement portion.

According to the invention of claim 3, the cell supply channel is not aligned with the corresponding cell drain channel connected by the reservoir. This results in the temporal storage of the cell in the reservoir which leads to accurate and easy cell transfer manipulation to the cell arrangement portion from the reservoir.

According to the invention of claim 4, plural of reservoirs and a single cell arrangement portion are provided. Each reservoir comprises a different cell supply channel, a different cell drain channel and a different junction channel from the other reservoir. Each junction channel connects each of reservoirs to the single cell arrangement portion. This configuration makes it possible to deliver various types of cells to the cell arrangement portion without mixing them in the channel, so that it is possible to identify the cell type without fluorescent label and so on even if handling different cells with similar shape or size.

According to the invention of claim 5, the cell arrangement portion comprises the compartments in a hexagonal shape so as to be arranged in a honeycomb structure so that it is suitable for a number of cells to be regularly-aligned. Also, the bottom of each compartment includes a circular aperture smaller than the hexagonal shape so that the cells can be fixed therein firmly.

The invention of claim 6 provides quick and accurate cell transfer and arrangement through the following three steps: a first step of introducing a liquid containing the cells from the supply mechanism into the at least one reservoir via the cell supply channel, a second step of transferring the cells from the reservoir to the cell arrangement portion via the junction channel with a control of the supply mechanism and the drain mechanism or the optical tweezer, and a third step of placing the cells into the compartments desirably selected in the reservoir with the optical tweezer. Thus, the cell chip can be manufactured efficiently and accurately.

According to the invention of claim 7, plural of cell supply channels, reservoirs and junction channels are provided. Each reservoir receives different types of cells through the corresponding cell supply channel than the other reservoirs. Desired cells selected from the cells located at each reservoir delivered to a cell arrangement portion without mixing the different types of cells in the channel. Therefore, it is possible to accurately and quickly fabricate a cell chip in which different types of cells are arranged without fluorescent label and so on.

### DESCRIPTION OF THE PREFERRED EMBODIMENTS

Hereinafter, preferred embodiments of a microchip and a method for cell arranging according to the present invention will be described with reference to the drawings.

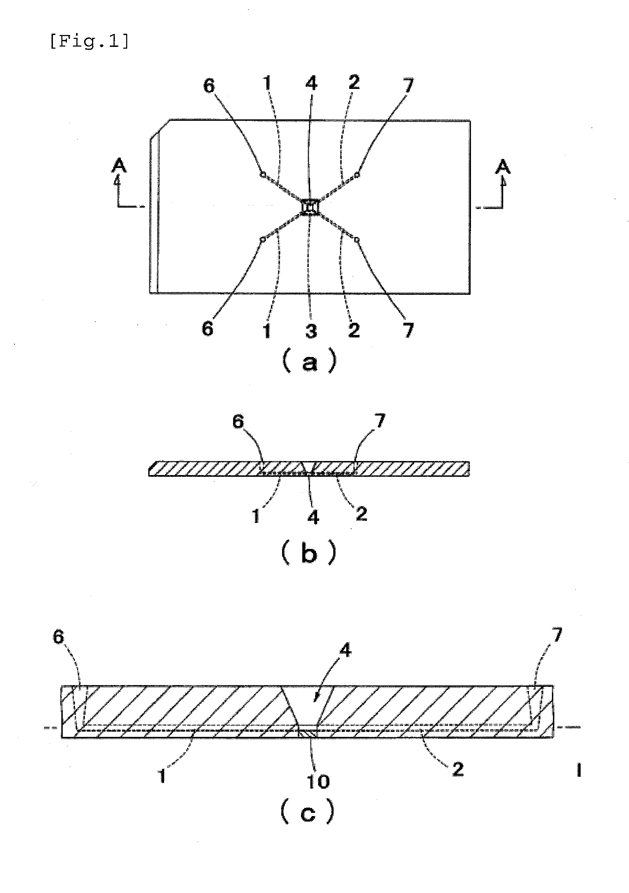

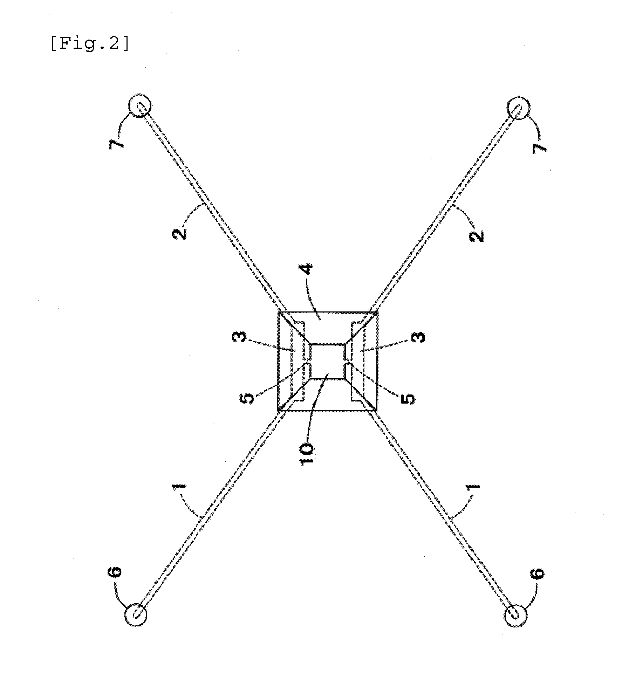

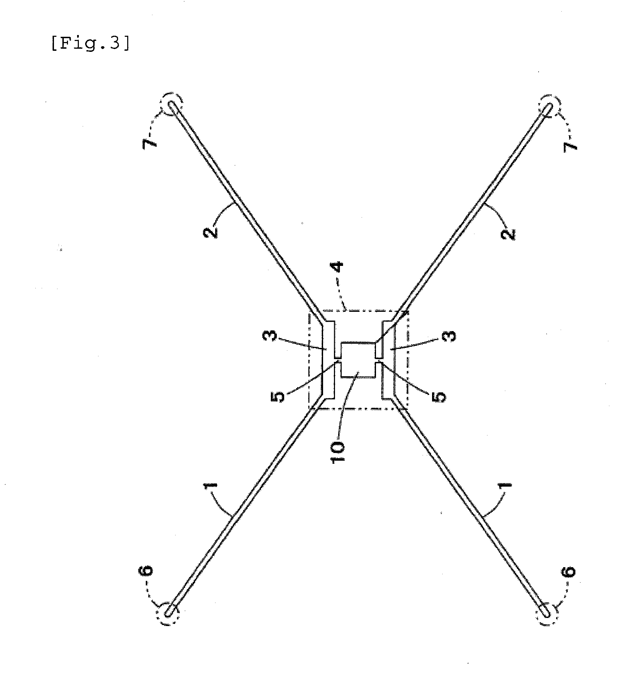

Fig. 1 shows the microchip for arranging cells (hereinafter simply referred as microchip) according to the present invention. Fig. 1 (a) is a plane view, Fig. 1 (b) is a cross-sectional view along A-A line shown in Fig.1 (a) and Fig. 1 (c) is an enlarged view around the center of Fig. 1 (b). Fig. 2 is an enlarged view around the center of Fig. 1 (a) . Fig. 3 is a cross-sectional view along B-B line shown in Fig. 1 (c).

The microchip of this invention includes an approximately flat base formed with a synthetic resin with optical transparency such as a light curing resin. The base comprises at least one cell supply channel 1, at least one cell drain channel 2, at least one reservoir 3, at least one cell arrangement portion 4 and at least one junction channel 5.

The cell supply channel 1 for transferring cells delivered with a liquid from a supply mechanism into the reservoir 3 is a hollow channel about 100µm in an inner diameter buried inside the base so that it does not appear on the outer surface of the base.

The cell supply channel 1 is formed with a cell inlet port 6 in liquid communication with a supply mechanism (not shown) such as a micropump or a microsyringe and so on at its start point. It is also connected to a cell drain channel 2 via a reservoir 3 at its end point.

The cell drain channel 2 for draining cells from the reservoir 3 by suction of a drain mechanism is a hollow channel about 100µm in an inner diameter buried inside the base so that it does not appear on the outer surface of the base similar to the cell supply channel 1.

The cell drain channel 2 is formed with a cell drain port 7 in liquid communication with a drain mechanism (not shown) such as a micropump or a microsyringe and so on at its end point. It is also connected to a cell supply channel 1 via a reservoir 3 at its start point.

The reservoir 3 is located at a common middle point of the cell supply channel 1 and the cell drain channel 2 to connect them inside the base. The reservoir 3 works for a temporal storage of the cells flowing to the cell drain channel 2 from the cell supply channel 1.

The reservoir 3 is wider than the cell supply channel 1 and the cell drain channel 2 (for example, more than twice) . Thus, the flow of the liquid containing the cells via the cell supply channel 1 is decelerated at the reservoir 3. This prevents the liquid from flowing into the cell drain channel 2.

The cell arrangement portion 4 is connected to the reservoir 3 via the junction channel 5 so that the cell arrangement portion 4 receives the cells introduced from the reservoir 3 for cell arrangement manipulation therein.

The cell arrangement portion 4 is a quadrangular room of which upper portion is formed in a truncated pyramid tapered downwardly. The upper edge of the cell arrangement portion defines an opening on the outer surface of the base. Optionally the opening may be covered with a lid (not shown) to provide an open condition for ventilation and a closed condition for preventing evaporation of solution during a long cell culture. For a short cell culture, the cell arrangement portion 4 may be buried inside the base similarly to the cell supply channel 1, the cell drain channel 2 and the reservoir 3.

The cell arrangement portion 4 includes plural of cell compartments for storing the cell partitioned by walls.

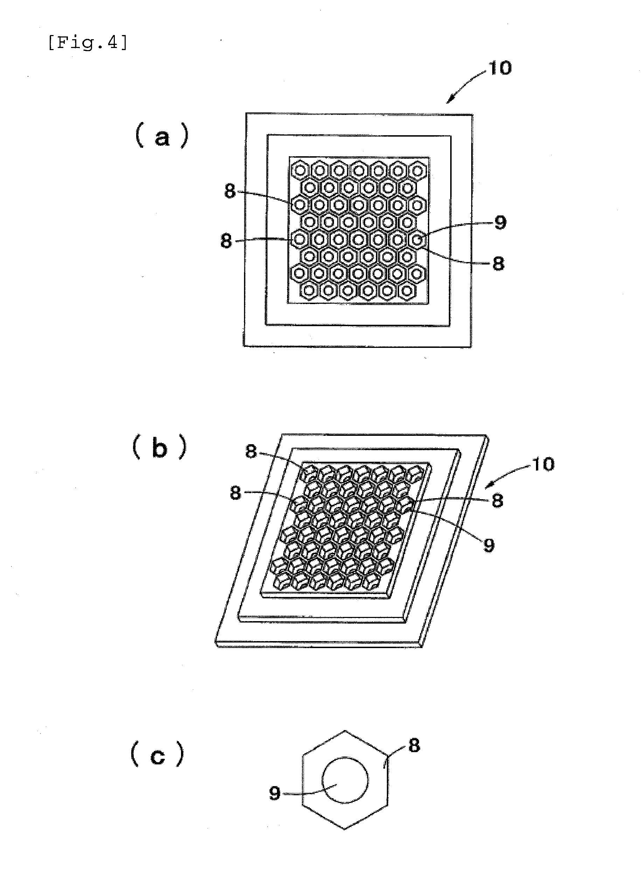

Fig.4 shows a micro base 10 including the cell compartments for storing the cell in the cell arrangement portion 4.

As shown in Fig.1 (c), the micro base 10 is integrally fitted to the bottom of the microchip base. Thus, the upper surface of the micro base defines a bottom surface of the cell arrangement portion 4.

There are a number of cell compartments 8 formed on the upper surface of the micro base 10.

A number of cell compartments 8 with hexagonal shape are arranged in a honeycomb structure on the micro base 10. Such honeycomb structure is suitable any regular arrangement for many cells.

A circular aperture 9 is formed on the bottom of the cell compartment 8. The circular aperture 9 is smaller than an outer shape of the hexagonal shape, more specifically, smaller than an inscribed circle of the hexagonal shape.

The circular aperture 9 on the bottom of the hexagonal cell compartment 8 facilitates to stably fix a cell at the center of the compartment 8 without excentric positioning resulting from cell characteristics that a cell tends to adhere any inner wall surface of the compartment 8 when a cell is placed in the hexagonal compartments.

It should be noted that the structure of cell arrangement portion 4 according to the present invention is not limited to the hexagonal cell compartment 8 arranged in the honeycomb pattern in Fig.4.

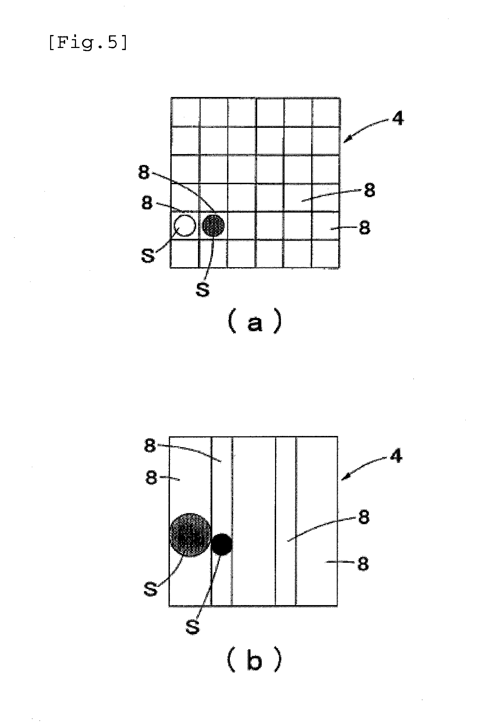

The other structures can be exemplified such as; the square cell compartments 8 arranged in a reticular pattern (shown in Fig.5(a)), and the straight grooved cell compartments 8 arranged in parallel (shown in Fig.5 (b)). In Fig.5, (S) represents a cell. As shown in Fig.3 (b), different sizes of cells can be stored by changing a width of the groove. This can be preferably used in detecting an interaction between the cells.

The junction channel 5 connected to the reservoir 3 and the cell arrangement portion 4. The junction channel 5 is narrower than the reservoir 3 (preferably about 1/2 to 1/3).

An angle (θ) at a corner of a flow path from the cell supply channel 1 to the junction channel 5 via the reservoir 3 is a right angle or an acute angle.

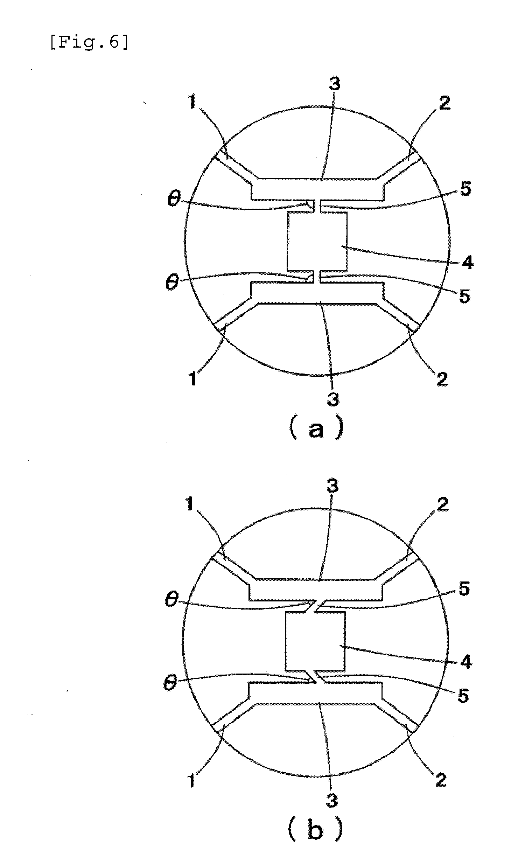

Fig.6(a) shows an example with the right angle, and Fig.6(b) with an acute angle.
Such dimensions of the width and the angle of the junction channel 5 may prevent the number of cells from flowing into the cell arrangement portion 4 from the reservoir 3 via the junction channel 5. Therefore, excessive cells do not flow into the cell arrangement portion 4.

The cell supply channel 1 is connected with the cell drain channel 2 via the reservoir 3 but not align with each other.

This configuration surely provides a temporal storage of the cells in the reservoir 3 when the cells through the supply channel 1 reach the reservoir 3. Thus, the cell transfer from the reservoir to the cell arrangement portion 4 may be easily operated.

The microchip according to the present invention, a number of reservoirs 3 are connected to one cell arrangement portion 4 via separate junction channels 5. Two reservoirs 3 are symmetrically disposed with respect to the cell arrangement portion 4 in the drawing, however, more than three reservoirs can be also provided.

Each reservoir 3 is connected to a different cell supply channel 1 and cell drain channel 2, respectively. That is, a number of channels are provided for connecting the cell supply channel 1, the reservoir 3 and cell drain channel 2, and these numbers of channels are symmetrically disposed with respect to the cell arrangement portion 4.

This configuration makes it possible to transfer the cells to the cell arrangement portion 4 without mixing different types of cells, so that any fluorescent label and so on may not be required.

Next, the cell arranging method of this invention will be explained.

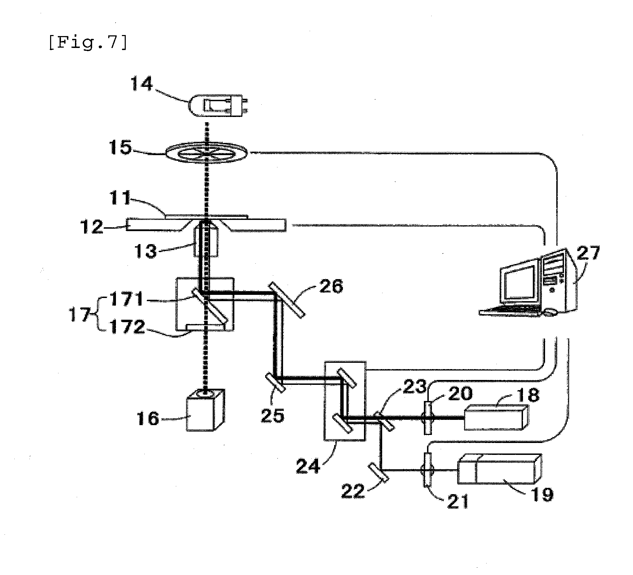

Fig. 7 is a schematic view showing one example of a device used in the cell arranging method of this invention. The device consists of an inverted microscope combined with a laser source and so on. Now, a structure of this device will be explained.

The microscope includes an electric stage 12 capable of moving along an X-Y axes and supporting the microchip 11, an objective lens 13 placed directly below the electric stage 12 to focus a light from the laser source (to be described later) and guide the light to the microchip 11, a laser source lamp 14 with a halogen lamp placed vertically above the objective lens 13 to send the light to the microchip 11, an electric shutter 15 placed between the laser source lamp 14 and the microchip 11 to control a quantity of light from the laser source lamp 14 to the microchip 11, and an imaging device 16 such as a CCD camera or CMOS camera which captures a transmission image of a visible light from the laser source lamp 14 passing the microchip 11.

Movement of the electric stage 12 and opening /closing operation of the electric shutter 15 are controlled by a control signal from a control software of a computer 27.

A mirror unit 17 comprising a dichroic mirror 171 and an absorbing filter 172 is placed vertically below the objective lens 13.

The dichroic mirror 171 changes a light path from the laser source toward the objective lens 13. The dichroic mirror 171 also passes the light from the laser source lamp 14 and guides it to the imaging device 16.

The absorbing filter 172 passes only the visible light components selectively from light components with various wavelengths from the laser source lamp 14 after the dichroic mirror 171.

The laser source comprises a first laser source 18 which emits an IR laser and a second laser source 19 which emits a UV laser.

The IR laser from the first laser source 18 is used as a trapping laser to trap and control the cell, i.e., an optical tweezer. For example, YAG laser (wavelength of 1060nm), Nd:YLF laser (wavelength 1047nm), DPSS laser (wavelength of 1064nm) and so on can be used as the IR laser, but not limited to these lasers as long as it is controlled without any damages to cells.

The UV laser from the second laser source 19 is used as a cell fusion laser. However, the second laser source 19 is not always necessary for the method of the invention.

Electric shutters 20,21, dichroic mirrors 22,23,25,26 and electric mirror unit 24 are placed along an optical guiding path which leads the laser lights from the first laser source 18 and the second laser source 19 to the above-described dichroic mirror 171 of the mirror unit 17.

Electric shutters 20,21 are placed in front of exit apertures of the first laser source 18 and the second laser source 19, respectively.

They can be opened and closed independently by the control signal from the computer 27. Thus the laser lights from the first laser source 18 and the second laser source 19 can be led to the mirror unit 17 selectively via the electric mirror unit 24 and so on.

The dichroic mirror 23 is placed in front of the electric shutter 20, and the dichroic mirror 22 is placed in front of the electric shutter 21.

The dichroic mirror 22 changes laser light path from the second laser source 19 toward the dichroic mirror 23.

The dichroic mirror 23 passes the laser light from the first laser source 18 to the electric mirror unit 24. It also reflects the laser light from the second laser source 19 coming from dichroic mirror 22 and leads it to the electric mirror unit 24.

Therefore, the laser lights from the first laser source 18 and the second laser source 19 are led to the electric mirror unit 24 along the same path by the above-described dichroic mirrors 22,23.

The electric mirror unit 24 has two electric control mirrors which are controllable independently by the control signal from the computer 27. One mirror scans in the x-axis direction and the other in the y-axis direction when the scanning is performed in the microchip 11 on the electric stage 12.

A galvanometer mirror, a piezo-driven mirror, an actuator-driven mirror and so on are applicable to the electric control mirror.

The dichroic mirror 25 changes laser light path after the electric mirror unit 24 to the dichroic mirror 26.

The dichroic mirror 26 changes this laser light path toward the dichroic mirror 171 of the mirror unit 17.

The method for arranging cell according to the present invention is accomplished by using the above-mentioned device as below.

As a preparation, the microchip of the present invention described above is fixed on the electric stage 12 of the invert microscope. The supply mechanism and the drain mechanism such as a micro pump or a micro syringe and so on are prepared as well. Then, the supply mechanism is connected to the cell inlet port 6 of the microchip 11 and the drain mechanism is connected to the cell drain port 7 via a tube.

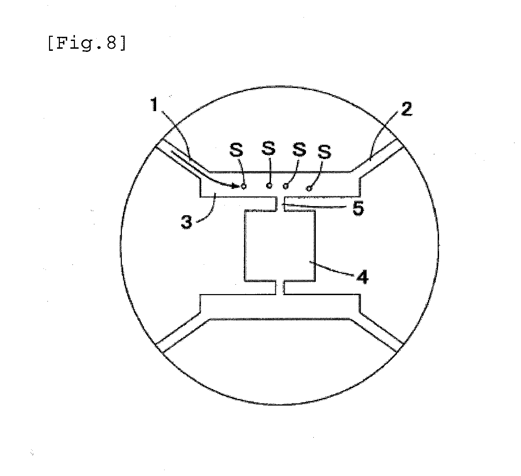

As the first step, the supply mechanism sends the liquid containing cells (S) into the reservoir 3 via the cell supply port 1 (Fig. 8). The liquid supply from the supply mechanism results in quick cell supply into the reservoir 3.

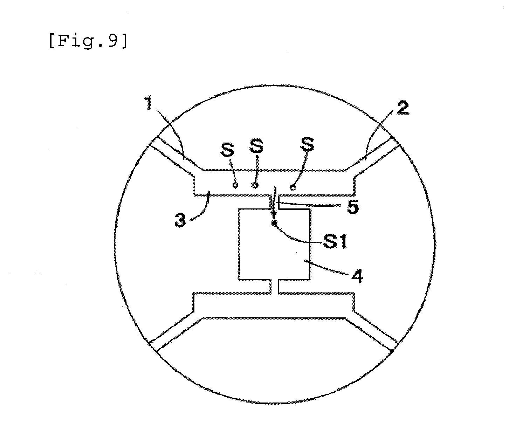

As the second step, the desired cell (S1) sent into the reservoir 3 is transferred to the cell arrangement portion 4 by controlling the supply mechanism and the drain mechanism or by using the optical tweezer (Fig. 9).

During the operation of the supply mechanism and the drain mechanism, the control to them set the flow rate sucked by the drain mechanism at a lower level than the flow rate pumped by the supply mechanism (the drain mechanism can stop its sucking operation).

When these flow rates are substantially equivalent, a liquid flow from the cell inlet port 6 to the cell supply channel becomes a laminar flow in the reservoir 3, and then goes through the cell drain channel 2. The control described above prevents the liquid introduced in the cell supply channel 1 via a cell inlet port 6 from smooth flow communication to the cell drain channel 2, which results in a liquid dispersion in the reservoir 3 so that the liquid flows into the cell arrangement portion 4 via the junction channel 5.

This control becomes more capable of transferring only the desired cells (S1) to the cell arrangement portion 4 via the junction channel 5 by introducing less cells into the cell supply channel 1.

In the case of the optical tweezer, the IR laser outputted from the first laser source 8 is guided to the reservoir 3 to trap the cell in the liquid, and then the laser moves by driving the electric mirror unit 24 so as to transfer the desired cell (S1) to the cell arrangement portion 4 via the junction channel 5.

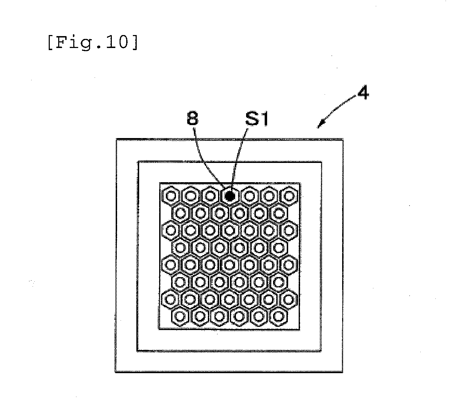

Finally, as the third step, the desired cell (S1) transferred into the cell arrangement portion 4 is placed into the circular aperture 9 of the desired cell compartment 8 (Fig.10) with applying the above-mentioned cell transfer manipulation of the optical tweezer.

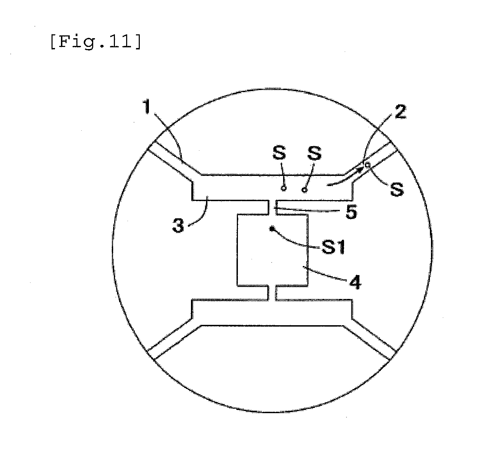

The supply mechanism and the drain mechanism is controlled so that the flow rate sucked by the drain mechanism is higher than the flow rate pumped by the supply mechanism (the supply mechanism can stop its pumping operation) to guide excessive cells contained in the liquid, which delivered from the cell supply channel 1 into the reservoir 3, toward the cell drain channel 2, and then the excessive cells exit from the cell drain port 7 (Fig.11).

It is possible to desirably arrange the cells in the cell arrangement portion 4 by repeating the first to the third steps as required.

It is possible to handle various types of the cells. In the above-described first step, each of the cell supply channel 1 receives a different type of cell which is then guided into each of the separate reservoirs 3.

As the above-described second step, each type of the desired cells selected from each of the separate reservoirs 3 is transferred into the cell arrangement portion 4 via each of the separate junction channels 5 by controlling the above-described supply mechanism and the drain mechanism or by the optical tweezer.

As the third step, the cells in the cell arrangement portion 4 are placed into the desired cell compartments with the optical tweezer.

Repetition from the first step to the third step results in a desired arrangement in the cell arrangement portion 4 with the various types of the cells.

### Industrial Utilization

The present invention is preferably applicable to fabrication of a cell chip to form a pseudo-tissue for toxic tests in a pharmaceutical production or a regeneration medicine or applicable to study for a cell manipulation such as a cell transfer, cell fusion, cell division or cell growth.

### Brief Description of Drawings

Fig. 1 shows the microchip for arranging cells according to the present invention. Fig.1(a) is a plane view, Fig. 1(b) is a cross-sectional view along A-A line shown in Fig.1(a) and Fig. 1(c) is an enlarged view of the center of Fig. 1(b).
Fig. 2 is an enlarged view around the center of Fig. 1 (a) .
Fig. 3 is a cross-sectional view along B-B line shown in Fig. 1 (c).
Fig. 4 shows a micro base including cell compartments. Fig 4 (a) is a plane view, Fig. 4 (b) is a perspective view and Fig. 4 (c) is an enlarged view for one of the compartments.
Fig. 5 shows another embodiment of the structure of the cell arrangement portion.
Fig. 6 explains an angle at a corner of a flow path from the cell supply channel to the junction channel. Fig. 6 (a) shows one embodiment with a right angle. Fig. 6 (b) shows the other embodiment with an acute angle.
Fig. 7 is a schematic view of the device used in the method for arranging the cells according to the present invention.
Fig. 8 shows the first step of the method for arranging the cells according to the present invention.
Fig. 9 shows the second step of the method for arranging the cells according to the present invention.
Fig. 10 shows the third step of the method for arranging the cells according to the present invention.
Fig. 11 shows removal of excessive cells in the method for arranging the cells according to the present invention.

### Explanation on the numeral

1 Cell supply channel
2 Cell drain channel
3 Reservoir
4 Cell arrangement portion
5 Junction channel
6 Cell inlet port
7 Cell drain port
8 Cell compartment
9 Circular aperture
11 Microchip
S Cell
S1 Desired cell

## Claims

1. A microchip for arranging cells, including a base, the base comprising:
at least one cell supply channel including a cell inlet port in liquid communication with a supply mechanism configured to supply the cells into the cell supply channel;
at least one cell drain channel including a cell drain port in fluid communication with a drain mechanism, the cell drain channel configured to guide the cells to the cell drain port;
at least one reservoir for temporarily storing the cells flowing between the cell supply channel and the cell drain channel;
at least one cell arrangement portion for receiving cells introduced from the reservoir, the cell arrangement portion comprising plural compartments partitioned by a wall; and
at least one junction channel extending from the cell arrangement portion to the reservoir;
wherein the reservoir is wider than the cell supply channel, the cell drain channel and the junction channel.

2. The microchip according to Claim 1, wherein an angle at a corner of a flow path through the reservoir from the cell supply channel to the junction channel is a right angle or an acute angle.

3. The microchip according to Claim 1 or 2, wherein the cell supply channel is not aligned with the cell drain channel connected to the cell supply channel via the reservoir.

4. The microchip according to any one of Claims 1 to 3 comprising plural of the reservoirs, each of reservoir connected to a separate cell supply channel, a separate cell drain channel and a separate junction channel,
wherein the separate junction channel terminating at the cell arrangement portion.

5. The microchip according to any one of Claims 1 to 4, comprising the compartments in a hexagonal shape so as to be arranged in a honeycomb structure,
wherein a bottom of each compartment includes a circular aperture smaller than the hexagonal shape.

6. A method for arranging cells with the microchip described in any one of Claims 1 to 5, comprising:
sequentially repeating a first step of introducing a liquid containing the cells from the supply mechanism into the at least one reservoir via the cell supply channel, a second step of transferring the cells from the reservoir to the cell arrangement portion via the junction with a control of the supply mechanism and the drain mechanism or with an optical tweezer, and a third step of placing the cells into the compartments desirably selected in the reservoir with the optical tweezer until a completion of a desired cell arrangement in the cell arrangement portion.

7. The method according to Claim 6, further comprising:
providing the microchip described in Claim 4 or 5, and
providing different types of the cells,
wherein
the first step includes introducing the cells with sorting by their types so that each reservoir receives different types of cells, and
the second step includes transferring a desired cell from each reservoir to the cell arrangement portion via the separate junction channel by controlling the supply mechanism and the drain mechanism or by the optical tweezer.
